# EUROPÄISCHE PATENTSCHRIFT

(11) **EP 3 568 056 B1**
(45) Veröffentlichungstag und Bekanntmachung des Hinweises auf die Patenterteilung: **05.08.2026**
(21) Anmeldenummer: 18703898.9
(22) Anmeldetag: 09.01.2018
(51) Int. Cl.: A61B 1/253, A61C 17/06

(54) **EINSTÜCKIGER SPIEGELSAUGER**
SINGLE-PIECE SUCTION MIRROR
SYSTÈME D'ASPIRATION À MIROIR D'UN SEUL TENANT

(30) Priorität: 10.01.2017 DE 102017000106
(43) Veröffentlichungstag der Anmeldung: 20.11.2019
(73) Patentinhaber: Cleverdent Ltd., 48149 Münster (DE)
(72) Erfinder: CLASEN, Stephan, 48149 Münster (DE); KAYSER, Martin, 50968 Köln (DE)
(74) Vertreter: Patentanwälte Bauer Vorberg Kayser
(86) Internationale Anmeldenummer: PCT/EP2018/050478
(87) Internationale Veröffentlichungsnummer: WO 2018/130529

(56) Entgegenhaltungen:
- EP-A1- 0 984 738
- DE-A1- 102013 110 302
- US-A- 3 092 910
- US-A- 3 928 916

## Beschreibung

Die vorliegende Erfindung betrifft einen zahnmedizinischen Spiegelsauger gemäß des Anspruchs 1. Weiterhin betrifft die Erfindung ein Verfahren zur Herstellung eines solchen Spiegelsaugers gemäß des Anspruchs 7.

Bei zahnmedizinischen Behandlungen ist es oftmals notwendig, anfallende Flüssigkeiten oder gelöste Partikel, beispielsweise Speichel, Spraywasser und Blut während der Behandlung abzusaugen. Auch kann Wasser, beispielsweise zum Reinigen oder nach Anwenden einer Multifunktionsspritze anfallen, das abgesaugt werden muss. Hierzu werden üblicherweise Absauger verwendet, die in der Regel aus einem röhrenförmigen Körper aus Kunststoff gebildet sind, an dessen Ende ein Schlauch befestigt ist, der wiederum mit einer Pumpe verbunden ist. Durch den Schlauch werden die störenden Flüssigkeiten und Festkörper abgeleitet.

Oftmals wird ein Absauger nicht vom behandelnden Zahnarzt oder Zahnchirurgen selbst, sondern von einer oder einem Assistenten geführt und gehalten, weil der behandelnde Zahnarzt mit der einen Hand ein Bohrwerkzeug und mit der anderen Hand einen Spiegel, über den er den zu behandelnden Bereich einsehen kann, halten muss. Nachteilig bei der beschrieben Vorgehensweise ist, dass die beiden Personen sehr eng beieinander um den zu behandelnden Bereich stehen oder sitzen müssen. Dies kann gerade dann, wenn es sich um verhältnismäßig schwierige oder feinmotorisch anspruchsvolle Eingriffe handelt, für den behandelnden Arzt als störend empfunden werden.

Aus der DE 10 2006 048 463 A1 ist ein medizinischer Spiegelsauger bekannt, bei dem die Innenfläche eine durch die Ansaugöffnung sichtbare verspiegelte Oberfläche aufweist. Die erfindungsgemäße spiegelnde Beschichtung ermöglicht es dem Benutzer, den medizinischen Spiegelsauger sowohl als Spiegelsauger zum Abführen von Flüssigkeiten und Partikeln, als auch gleichzeitig als Spiegel zu benutzen. Mit Hilfe eines solchen Spiegelsaugers ist es ihm nun möglich, die Behandlung ohne eine assistierende Person durchzuführen. Der Spiegelsauger wird also gleichzeitig als Absauger und als Spiegel verwendet.

Die Grundidee der Kombination der beiden Geräte ist grundsätzlich sehr gut, allerdings hat sich die praktische Umsetzung der Idee bzw. die Herstellung eines solchen Spiegelsaugers als äußerst schwierig herausgestellt. Der Spiegel muss fest eingefasst sein, er darf sich nicht lösen und sollte sich auch während der Behandlung nicht bewegen dürfen, da dadurch die Sicht beeinträchtigt wird. Auch dürfen möglichst keine Unebenheiten oder Spalte vorhanden sein, da diese die Geräuschentwicklung negativ beeinflussen.

Der Grundkörper des Spiegelsaugers kann beispielsweise aus zwei verschweißten Längshälften gebildet sein, die den Spiegel in einer Nut halten. Ein spaltfreies Verschweißen der Längshälften ist aber kaum zufriedenstellend möglich, es wird entweder eine Schattenfuge gebildet oder es entsteht beim Schweißvorgang Materialüberschuss, der anschließend abgetragen werden muss. Die Schattenfuge ist unschön und es können sich Flüssigkeiten und Bakterien in ihr sammeln, der Abtrag des Materialüberschusses ist zeitaufwändig und führt zu sichtbaren Veränderungen der Oberfläche des Grundkörpers. Die beiden Längshälften müssen darüber hinaus ausgesprochen exakt gefertigt sein, um nach dem Zusammensetzen bzw. Verschweißen eine ansprechende, insbesondere spalt- und/oder überschussfreie Erscheinung zu gewährleisten. Diese sind vor allem deswegen störend, weil der behandelnde Zahnarzt im Längsverlauf des Spiegelsaugers jede Unebenheit, Nut oder Grad mit den Fingern spürt. Insbesondere sind auch in Längsrichtung nur geringste Längentoleranzen der beiden Hälften akzeptabel, die beiden Hälften dürfen darüber hinaus in Längsrichtung auch nicht minimal versetzt zueinander angeordnet sein.

Aus der DE 10 2013 110 302 A1 ist ein ähnlicher medizinischer Spiegelsauger bekannt, der ebenfalls in einem relativ aufwändigen Verfahren aus zwei Grundkörperteilen gefertigt ist.

Die US 3 092 910 A offenbart einen Absauger, der ebenfalls einen Spiegel in seinem Frontbereich aufweist. Der Spiegel kann über einen zweiten Kanal mit einem Fluid freigeblasen werden. Das Fluid wird über den anderen Kanal wieder angesaugt und abgeleitet.

Die Aufgabe der vorliegenden Erfindung besteht darin, einen Spiegelsauger bereitzustellen, der eine ausreichend hochwertige Spiegelfläche im Bereich der Ansaugöffnung aufweist. Der Spiegelsauger soll dabei kostengünstig herstellbar sein und ein ansprechendes Äußeres aufweisen. Die Nachteile des Standes der Technik sollen vermieden werden, insbesondere soll der Spiegelsauger keine oder nur unwesentliche Spalte auf seiner Außenseite aufweisen. Weiterhin ist es Aufgabe der Erfindung, ein Verfahren zur Herstellung eines Spiegelsaugers mit den genannten Vorteilen vorzuschlagen.

Erfindungsgemäß wird die Aufgabe durch einen Spiegelsauger mit den Merkmalen des Patentanspruchs 1 gelöst. Ein erfindungsgemäßes Herstellungsverfahren ist in Patentanspruch 7 angegeben.

Demnach ist der Spiegelsauger aus nur einem einzigen Grundkörper gebildet. Der Grundkörper ist erfindungsgemäß in einem einzigen Verfahrensschritt im Spritzgussverfahren gefertigt.

Eine Spiegelaufnahme ist als Vertiefung oder Mulde ausgeführt, in die der Spiegel nach Fertigung des Grundkörpers in einem zweiten Verfahrensschritt schnell und einfach von oben eingedrückt wird. Dementsprechend weist die Spiegelaufnahme einen Hinterschnitt auf, der durch eine obere Halteschulter gebildet ist. Dementsprechend ist die Spiegelaufnahme bzw. die Halteschulter elastisch ausgeführt, sodass diese beim Eindrücken des Spiegels zurückweichen kann und der Spiegel in die Spielaufnahme eingeklipst werden kann.

Um das Einsetzen des Spiegels zu erleichtern, ist dieser vorzugsweise im Querschnitt konisch ausgeführt. Sein Durchmesser nimmt ausgehend von der Spiegelfläche in Richtung der Spielrückseite ab.

Der Spiegel ist nach dem Einsetzen fest im Spiegelsauger befestigt, was insofern vorteilhaft ist, weil der Grundkörper und der Spiegel im Praxisbetrieb als Einheit behandelt werden können. Der Spiegel und Spiegelsauger können gemeinsam sterilisiert werden. Der Begriff "fest" bedeutet in diesem Zusammenhang, dass der Spiegel unverlierbar gehalten ist, er kann nicht herausgelöst werden, ohne den Spiegelsauger oder den Spiegel zu zerstören.

Der Spiegel ist vorteilhafterweise rund, kann aber auch oval sein oder andere geeignete Formen aufweisen. Im Folgenden wird von der üblichen runden Form des Spiegels ausgegangen.

Das den Spiegel umgebende und ihn haltende Material ist erfindungsgemäß weich oder elastisch, so dass es Materialausdehnungen, die beispielsweise bei der Sterilisation entstehen ausgleichen kann. Ungewollte Spannungen im Bereich des Spiegels, die den Spiegel und/oder den Grundkörper zerstören können, werden dadurch wirkungsvoll vermieden. Zusätzlich kann der Spiegel in einer Nut gehalten sein, die in seitlicher Richtung ausreichend tief ist, um eine Wärmeausdehnung bzw. eine Vergrößerung der Fläche und des Durchmessers des Spiegels auszugleichen. Der sich ausdehnende Spiegel kann sich in die ausrechend tiefe Nut hinein erstrecken. Anstelle der Nut kann auch der Durchmesser der Öffnung im ersten Grundkörperteil, in die der Spiegel eingesetzt ist, einen etwas größeren Durchmesser als der Spiegel aufweisen.

Die Spiegeloberfläche und die umgebende Bodenfläche bilden eine möglichst plane Gesamtfläche, über die der Luftstrom, angesaugte Flüssigkeit und Partikel optimal abgeführt werden können. Die plane Gesamtfläche bewirkt auch, dass die Geräuschentwicklung durch Luftverwirbelungen in diesem Bereich gering ist. Ein Überstand des Spiegels gegenüber der Bodenfläche des ersten Grundkörperteils von bis zu 0,3 mm wird im Sinne der Erfindung noch als bündig angesehen.

Für die Fertigung eignet sich insbesondere thermoplastischer Kunststoff zum Beispiel Polypropylen oder auch Polyethylen. Durch die Hinzugabe von Zusatzstoffen kann die äußere Erscheinung des Spiegelsaugers beeinflusst werden. Erfindungsgemäß hat sich auch die Fertigung aus Polyester als vorteilhaft erwiesen. Polyester weist hinsichtlich der Oberflächenbeschaffenheit des fertigen Produkts gegenüber anderen Kunststoffen deutliche Vorteile beispielsweise hinsichtlich des einstellbaren Glanzgrades, der Kratzfestigkeit sowie der Oberflächenglätte auf. Als geeigneter Zuschlagstoff zur Beeinflussung der Materialeigenschaften kommen beispielsweise Glaskugeln in Frage, die unter anderem den Glanzgrad der Oberfläche beeinflussen.

Die Ansaugöffnung des rohrförmigen Grundkörpers verläuft nicht rechtwinklig zur Längsachse, sondern ist schräg zu ihr ausgeführt. Dadurch ergibt sich eine schräg zulaufende Form des Spiegelsaugers, wodurch dieser leichter beispielsweise zwischen Wange und Zähne einführbar ist. Der Spiegel befindet sich nicht vor der Ansaugöffnung, sondern in Strömungsrichtung der angesaugten Luft im Wesentlichen hinter der Ansaugöffnung, also innerhalb des Grundkörpers. Hierdurch wird erreicht, dass der Spiegelsauger nicht durch einen vorgelagerten Spiegel verlängert wird, was die Ansaugleistung vermindern würde.

Das erfindungsgemäße Verfahren zur Herstellung des Spiegelsaugers weist die folgenden Verfahrensschritte auf
- Fertigen eines einzigen Grundkörpers mit einer als Vertiefung ausgeführten Spiegelaufnahme für den Spiegel, die einen durch eine obere, eine gesamte Außenwandung des Spiegels umgebende und einen Raum neben und unterhalb des Spiegels abdichtende elastische Halteschulter gebildeten Hinterschnitt aufweist,
- Einsetzen des Spiegels von oben durch Eindrücken in die Spiegelaufnahme derart, dass die obere Halteschulter aufgrund ihrer Elastizität zunächst durch den Spiegel nach außen gedrückt wird und anschließend eine Oberseite des Spiegels kontaktiert und den Spiegel in der Spiegelaufnahme hält.

Die Erfindung wird anhand der nachfolgenden Figuren näher erläutert. Diese zeigen lediglich Ausführungsbeispiele, die Erfindung soll nicht auf diese beschränkt sein.

Es zeigen:
- Fig. 1:: einen erfindungsgemäßen Spiegelsauger von oben,
- Fig. 2:: den erfindungsgemäßen Spiegelsauger aus Fig. 1 von der Seite,
- Fig. 3:: den erfindungsgemäßen Spiegelsauger aus Fig. 1 im Längsschnitt,
- Fig. 4.:: eine Vergrößerung des Bereichs A aus Fig 3.
- Fig. 6:: eine vereinfachte Darstellung des vorderen Bereichs des Spiegelsau

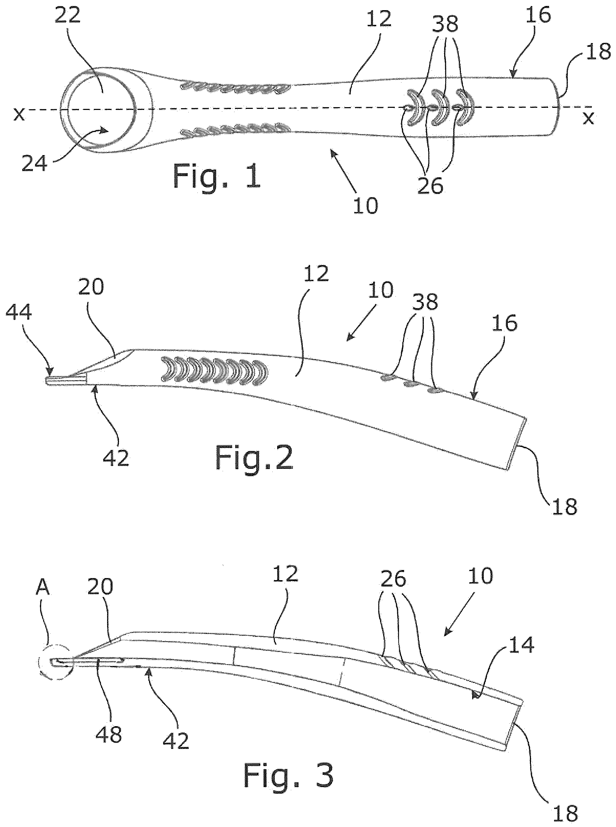

Wie sich insbesondere aus den Figuren 1 bis 3 ergibt, weist ein erfindungsgemäßer Spiegelsauger 10 einen hohlen rohrförmigen Grundkörper 12 mit einer Innenfläche 14 und einer Außenfläche 16 auf. Weiterhin weist der Grundkörper 12 eine Längsachse X-X auf (vergl. Fig. 1). Die insbesondere in den Figuren 2 und 3 erkennbare Bogenform des Spiegelsaugers 10 hat den Vorteil, dass dieser leichter an die zu behandelnde Stelle herangeführt werden kann.

Der Grundkörper 12 weist eine Anschlussöffnung 18 für einen nicht gezeigten Schlauch und eine Ansaugöffnung 20 zum Ansaugen von Partikeln und Flüssigkeiten auf. Durch die Ansaugöffnung 20 werden die abzusaugenden Flüssigkeiten oder Partikel eingesogen und durch die Anschlussöffnung 18 über den Schlauch abgeleitet.

Erfindungsgemäß ist innerhalb des Grundkörpers 12 ein Spiegel 22 im Bereich der Ansaugöffnung 20 angeordnet, der zumindest bereichsweise durch die Ansaugöffnung 20 einsehbar ist. Entsprechend ist eine sichtbare Spiegelfläche 24 der Ansaugöffnung 20 zugewandt. Der Spiegel 22 ist vorzugsweise vollständig innerhalb des Grundkörpers 12, also in Strömungsrichtung der abzusaugende in Luft hinter der Ansaugöffnung 20 angeordnet, er kam aber bereichsweise vor der Ansaugöffnung 20 angeordnet sein. Die angesaugte Luft wird über die Spiegelfläche 24 geleitet, wodurch ein Beschlagen der Spiegelfläche 24 effektiv verhindert wird.

Der Spiegelsauger 10 weist Zusatzöffnungen 26 auf, durch die ebenfalls Luft angesaugten wird. Die Zusatzöffnungen 26 verhindern einen Unterdruck innerhalb des Grundkörpers 12, wenn die Ansaugöffnung 20 beispielsweise durch die Zunge oder Wange des Patienten verschlossen wird. Im gezeigten Ausführungsbeispiel sind drei Zusatzöffnungen 26 vorgesehen, denkbar ist aber auch nur eine einzige Zusatzöffnung 26 oder auch mehr als drei Zusatzöffnungen 26.

Auf der Außenfläche 16 des Grundkörpers 12 sind Profilelemente 38 erkennbar, die einen sicheren Griff des Spiegelsaugers 10 und ein Abrutschen der Finger des behandelnden Zahnarztes verhindern.

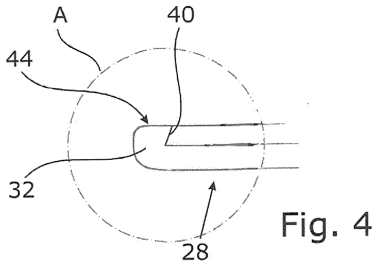

Die Figuren 3 und 4 zeigen eine Spiegelaufnahme 48, in die der Spiegel 22 im zusammengesetzten Zustand eingesetzt ist. Eine Innenwandung 30 einer Vertiefung 28 umgibt den Spiegel 22 und liegt an einer Außenwandung des Spiegels 22 zumindest bereichsweise an. Die Vertiefung 28 verjüngt sich ausgehend von einer Grundkörperunterseite 42 in Richtung der Ansaugöffnung 20 und bildet eine Halteschulter 40 aus.

Der Spiegel 22 kann im Querschnitt zumindest abschnittsweise auch trapezförmig ausgeführt sein, so dass sein Durchmesser zumindest abschnittsweise ausgehend von der Spiegelfläche 24 in Richtung einer Grundkörperunterseite 42 zunimmt. Diese Form vereinfacht das Einsetzen oder Einklipsen in die Spiegelaufnahme 48. Die Halteschulter 40 liegt in diesem Fall an dem sich in Richtung der Spiegelfläche 24 verjüngenden Außenumfang an.

Fig. 4 verdeutlicht in einer vergrößerten Darstellung des Bereichs A aus Fig. 4, dass erfindungsgemäß die obere Halteschulter 40 die gesamte Außenwandung 36 des Spiegels 22 umgibt, und einen Raum neben und unterhalb des Spiegels 22 abdichtet. Die Abdichtung wird über eine Vorspannung der oberen Halteschulter 40 verbessert. Dies bedeutet, dass der Spiegel 22 beim Einsetzen in das erste Grundkörperteil 32 gegen die obere Halteschulter 40 gedrückt wird und diese minimal komprimiert oder elastisch verformt wird.

## Patentansprüche

1. Zahnmedizinischer Spiegelsauger (10) zum Absaugen von Flüssigkeiten und Partikeln aus einem Mundraum eines Patienten, mit einem rohrförmigen hohlen Grundkörper (12), der eine Innenfläche (14), eine Außenfläche (16), eine Längsachse (X-X), eine Anschlussöffnung (18) für einen Schlauch und eine Ansaugöffnung (20) aufweist, wobei die Innenfläche (14) einen zumindest bereichsweise durch die Ansaugöffnung (20) einsehbaren Spiegel (22) mit einer der Ansaugöffnung (20) zugewandten sichtbaren Spiegelfläche (24) und einer Spiegelrückseite aufweist,
**dadurch gekennzeichnet, dass**
der Spiegelsauger (10) aus einem einzigen einstückig ausgeführten und in einem einzigen Verfahrensschritt im Spritzgussverfahren gefertigten Grundkörper (12) gebildet ist, der in seiner Innenfläche (14) im Bereich der Ansaugöffnung (20) eine als Vertiefung (28) ausgeführte Spiegelaufnahme (48) mit einem durch eine obere, eine gesamte Außenwandung (36) des Spiegels (22) umgebende und einen Raum neben und unterhalb des Spiegels (22) abdichtende elastische Halteschulter (40) gebildeten Hinterschnitt aufweist, sodass die obere Halteschulter (40) beim Eindrücken des Spiegels (22) von oben in Richtung einer Grundkörperunterseite (42) in die Vertiefung (28) zurückweichen kann, so dass der Spiegel (22) in die Spiegelaufnahme (48) eingeklipst und in dieser formschlüssig gehalten wird und wobei die Halteschulter (40) aufgrund ihrer Elastitzität Materialausdehnungen, die bei einer Sterilisation entstehen ausgleichen kann.

2. Zahnmedizinischer Spiegelsauger (10) nach Anspruch 1,
**dadurch gekennzeichnet, dass**
sich die Spiegelaufnahme (48) ausgehend von einer Grundkörperunterseite (42) in Richtung der Ansaugöffnung (20) verjüngt und eine Innenwandung (30) der Spiegelaufnahme (48) die Halteschulter (40) ausbildet, die eine Oberseite des Spiegels (22) kontaktiert und den Spiegel (22) in der Spiegelaufnahme (48) hält.

3. Zahnmedizinischer Spiegelsauger (10) nach Anspruch 2, **dadurch gekennzeichnet, dass** die Halteschulter (40) die Oberseite über den gesamten Umfang kontaktiert.

4. Zahnmedizinischer Spiegelsauger (10) nach einem der Ansprüche 1 bis 3, **dadurch gekennzeichnet, dass** der Spiegel (22) kreisförmig ausgebildet ist.

5. Zahnmedizinischer Spiegelsauger (10) nach einem der Ansprüche 1 bis 4, **dadurch gekennzeichnet, dass** der Spiegel (22) unverklebt gehalten ist.

6. Zahnmedizinischer Spiegelsauger (10) nach einem der Ansprüche 1 bis 5, **dadurch gekennzeichnet, dass** der Grundkörper (12) aus Kunststoff gefertigt ist.

7. Verfahren zur Herstellung eines zahnmedizinischen Spiegelsaugers (10) mit den Merkmalen der Ansprüche 1 bis 6, zum Absaugen von Flüssigkeiten und Partikeln aus einem Mundraum eines Patienten
**mit den Verfahrensschritten**
- Fertigen des einzigen Grundkörpers (12) in einem einzigen Verfahrensschritt im Spritzgussverfahren mit einer als Vertiefung (28) ausgeführten Spiegelaufnahme (48) für den Spiegel (22), die einen durch eine obere, eine gesamte Außenwandung (36) des Spiegels (22) umgebende und einen Raum neben und unterhalb des Spiegels (22) abdichtende elastische Halteschulter (40) gebildeten Hinterschnitt aufweist,
- Einsetzen des Spiegels (22) von oben in Richtung einer Grundkörperunterseite (42) durch Eindrücken in die Spiegelaufnahme (48) derart, dass die obere Halteschulter (40) aufgrund ihrer Elastizität zunächst durch den Spiegel (22) nach außen gedrückt wird und anschließend eine Oberseite des Spiegels (22) kontaktiert und den Spiegel (22) in der Spiegelaufnahme (48) hält.

8. Verfahren nach Anspruch 7, **dadurch gekennzeichnet, dass** der Spiegel im Querschnitt konisch zulaufend ausgeführt ist, wobei sich der Querschnitt ausgehend von der Spiegelfläche (24) in Richtung der Spiegelrückseite reduziert.

## Claims

1. A dental mirror suction device (10) for suctioning liquids and particles from a patient's oral cavity, comprising a tubular hollow body (12) having an inner surface (14), an outer surface (16), a longitudinal axis (X-X), a connection opening (18) for a hose, and a suction opening (20), wherein the inner surface (14) comprises a mirror (22) that is visible at least in part through the suction opening (20), with a visible mirror surface (24) facing the suction opening (20) and a mirror back surface, **characterized in that**
the mirror suction device (10) is formed from a single, one-piece base body (12) manufactured in a single step using an injection molding process, which has, in its inner surface (14) in the area of the suction opening (20), a mirror receptacle (28) designed as a recess (48) formed in its inner surface (14) in the region of the suction opening (20), which recess (48) comprises an undercut formed by an elastic retaining shoulder (40) surrounding an entire exterior wall (36) of the mirror (22) and sealing off a space adjacent to and beneath the mirror (22), such that the upper retaining shoulder (40), when the mirror (22) is pressed in from above toward a bottom surface of the base body (42) can retract into the recess (28), so that the mirror (22) is snapped into the mirror holder (48) and held therein by a form-fit, and wherein the retaining shoulder (40) can compensate for material expansion resulting from sterilization due to its elasticity.

2. Dental mirror suction device (10) according to claim 1,
**characterized in that**
the mirror receptacle (48) tapers from a bottom surface of the base body (42) toward the suction opening (20), and an inner wall (30) of the mirror receptacle (48) forms the retaining shoulder (40), which contacts an upper surface of the mirror (22) and holds the mirror (22) in the mirror receptacle (48).

3. A dental mirror suction device (10) according to claim 2, **characterized in that** the retaining sleeve (40) contacts the top surface along its entire circumference.

4. A dental mirror suction cup (10) according to any one of claims 1 to 3, **characterized in that** the mirror (22) is circular in shape.

5. A dental mirror suction device (10) according to any one of claims 1 to 4, **characterized in that** the mirror (22) is held in place without being glued.

6. A dental mirror suction cup (10) according to any one of claims 1 to 5, **characterized in that** the base body (12) is made of plastic.

7. Method for manufacturing a dental mirror suction device (10) having the features of claims 1 through 6, for extracting liquid liquids and particles from a patient's oral cavity **including the process steps**
- Manufacturing the single base body (12) in a single process step using an injection molding process, with a mirror receptacle (48) designed as a recess (28) for the mirror (22), which has an undercut formed by an upper retaining shoulder (40) that surrounds the entire outer wall (36) of the mirror (22) and seals a space adjacent to and beneath the mirror (22),
- inserting the mirror (22) from above toward a base body by pressing it into the mirror receptacle (48) such that the upper retaining shoulder (40), due to its elasticity, is initially pushed outward by the mirror (22) and subsequently contacts an upper surface of the mirror (22) and holds the mirror (22) in the mirror receptacle (48).

8. The method according to claim 7, **characterized in that** the mirror has a tapered cross-section, wherein the cross-section decreases from the mirror surface (24) toward the rear of the mirror.

## Revendications

1. Miroir dentaire aspirant (10) pour aspirer des liquides et des particules d'une cavité buccale d'un patient, comprenant un corps de base (12) creux en forme de tube qui présente une surface intérieure (14), une surface extérieure (16), un axe longitudinal (X-X), une ouverture de raccordement (18) pour un tuyau et une ouverture d'aspiration (20), dans lequel la surface intérieure (14) présente un miroir (22) visible au moins par tronçons à travers l'ouverture d'aspiration (20) avec une face de miroir (24) visible tournée vers l'ouverture d'aspiration (20), et un dos de miroir,
**caractérisé en ce que**
le miroir dentaire aspirant (10) est formé d'un corps de base (12) unique conçu d'un seul bloc et fabriqué au cours d'une seule étape de procédé de moulage par injection, qui présente dans sa surface intérieure (14) au niveau de l'ouverture d'aspiration (20), une réception de miroir (48) conçue en tant que renfoncement (28) avec une contre-dépouille formée par un épaulement de maintien (40) élastique supérieur entourant la totalité d'une paroi extérieure (36) du miroir (22) et étanchéifiant un espace près de et sous le miroir (22), de sorte que l'épaulement de maintien (40) supérieur, lorsque le miroir (22) est enfoncé par le haut, peut retourner en direction d'une sous-face (42) du corps de base dans le renfoncement (28), de sorte que le miroir (22) est clipsé dans la réception de miroir (48) et maintenu dans celle-ci par adhérence de forme, et dans lequel l'épaulement de maintien (40) peut compenser, du fait de son élasticité, des dilatations de matériau qui se produisent lors d'une stérilisation.

2. Miroir dentaire aspirant (10) selon la revendication 1,
**caractérisé en ce que**
la réception de miroir (48) se rétrécit en partant d'une sous-face (42) du corps de base en direction de l'ouverture d'aspiration (20) et une paroi intérieure (30) de la réception de miroir (48) forme l'épaulement de maintien (40), qui est en contact avec une face supérieure du miroir (22) et maintient le miroir (22) dans la réception de miroir (48).

3. Miroir dentaire aspirant (10) selon la revendication 2, **caractérisé en ce que** l'épaulement de maintien (40) est en contact avec la surface supérieure sur tout le pourtour.

4. Miroir dentaire aspirant (10) selon l'une des revendications 1 à 3, **caractérisé en ce que** le miroir (22) est circulaire.

5. Miroir dentaire aspirant (10) selon l'une des revendications 1 à 4, **caractérisé en ce que** le miroir (22) est maintenu non collé.

6. Miroir dentaire aspirant (10) selon l'une des revendications 1 à 5, **caractérisé en ce que** le corps de base (12) est fabriqué en plastique.

7. Procédé de fabrication d'un miroir dentaire aspirant (10) comprenant les caractéristiques des revendications 1 à 6, pour aspirer des liquides et des particules d'une cavité buccale d'un patient, comprenant les étapes de procédé de
- fabrication du corps de base (12) unique au cours d'une seule étape de procédé de moulage par injection, comprenant une réception de miroir (48) pour le miroir (22) conçue en tant que renfoncement (28) qui présente une contre-dépouille formée par un épaulement de maintien (40) élastique supérieur entourant la totalité d'une paroi extérieure (36) du miroir (22) et étanchéifiant un espace près de et sous le miroir (22),
- insertion du miroir (22) par le haut en direction d'une sous-face (42) du corps de base par enfoncement dans la réception de miroir (48) de telle façon que l'épaulement de maintien (40) supérieur, du fait de son élasticité, est d'abord appuyé vers l'extérieur par le miroir (22) et est enfin en contact avec une face supérieure du miroir (22) et maintient le miroir (22) dans la réception de miroir (48).

8. Procédé selon la revendication 7, **caractérisé en ce que** le miroir est conçu conique en section, dans lequel la section se réduit en partant de la surface de miroir (24) vers le dos du miroir.
